# EUROPEAN PATENT SPECIFICATION

(11) **EP 1 043 003 B1**
(45) Date of publication and mention of the grant of the patent: **19.05.2004**
(21) Application number: 00302346.2
(22) Date of filing: 22.03.2000
(51) Int. Cl.: A61F 2/72

(54) **Prosthetic limbs**
Gliederprothesen
Membres prothétiques

(30) Priority: 22.03.1999 GB 9906604; 14.04.1999 GB 9908551
(43) Date of publication of application: 11.10.2000
(73) Proprietor: Advanced Control Research Ltd., Plymton, Plymouth PL7 2AW (GB)
(72) Inventor: Burns, Roland Stephen, Plympton, Plymouth PL7 2DH (GB); Nurse, Peter, Blackawton, Nr. Totnes TQ9 7DL (GB)
(74) Representative: Harrison, Ivor Stanley

(56) References cited:
- EP-A- 0 421 780
- US-A- 4 209 860
- US-A- 5 413 611

## Description

This invention relates to prosthetic limbs and especially to prosthetic limbs having distal free digitate appendages such as prosthetic hands.

Prosthetic hands for arm amputees have been available for several decades but until the 1970's were generally cable-operated. More recently, a prosthetic hand has been developed in which the thumb and coupled index and middle fingers cooperate in a single degree of freedom pincer-like action, where the action is controlled by sensing the myoeletric signals (MES) generated at the skin surface of the stump when the brain sends a "muscle contract" command. For example, two MES sensors may be applied to the skin surface below the elbow and, when the sensed MES exceed a specified threshold, the thumb and coupled finger open or close. A prosthetic limb responding to MES is known from EP-A-421 780.

Further work on MES has established that the amplitude and frequency thereof vary for different hand movements for a given muscle site, and that the MES signature for a particular movement is reasonably consistent and repeatable. It is an object of the present invention to provide a prosthetic limb, especially an arm, where the prosthetic hand attached thereto can perform more realistic digit movements than have hitherto been possible.

According to one aspect of the present invention, there is provided a prosthetic limb system according to the subject-matter of claim 1.

According to another aspect, the invention provides a method for controlling a prosthetic limb, according to the subject-matter of claim 6.

Preferably, the prosthetic limb comprises a prosthetic hand and the elements thereof are an artificial thumb and at least one artificial finger.

The sensing means are preferably disposed at selected zones of the skin of the stump, according to the limb movement which it is intended to provide, since it is known that myoelectric signals reflecting or appertaining to particular limb movements and tissue filter functions occur at discrete sites on the upper limb.

The sensed signals are amplified and normalised and are preferably filtered and classified according to their frequency content in a perception subsystem. The signals are converted from the time to the frequency domain by the use of bandpass filters operating within specified frequency ranges between 0 and 1000 Hz, preferably 0 - 400 Hz, and processed to calculate the RMS value of each filter output, preferably with performance standards for speed of recognition (e.g. <0.4 seconds), percentage of non-recognitions (e.g. <5%) and percentage of mis-recognitions (e.g: <0.5%). The artificial neural network may comprise interconnected input, hidden and output layers and can be trained to recognise and react to specific patterns; the outputs are associated with discrete limb positions. Preferably, a back-propagation learning algorithm is utilised to train the logic base of the neural network, preferably modified with added momentum and learning coefficients. A neural network with three binary output signals would provide up to eight desired hand positions. The decoded myoelectric signals from the neural network are passed to a fuzzy logic classifier in order to provide a definitive desired hand position. This is then passed to a cognition system which will compare the actual finger positions with a suite of stored desired finger positions, any difference existing being used to actuate the fingers until the difference is zero. Significant performance criteria would include stability over all operating regimes and a fast response time with no overshoot; desirably the settling time should be short, (e.g. <0.8 seconds). All processing calculations including digital filtering by the use of bandpass filters, neural network pattern recognition and post-processing can be undertaken within one sampling period, typically one-thousandth of a second.

The servo-drive means preferably, for a prosthetic hand, will operate five active fingers in as many as eleven degrees of freedom of movement within performance criteria e.g. size of actuator, range of movement, resolution and repeatability, linearity, force to signal ratio, quietness of operation and power consumption.

Embodiments of the invention will now be described by way of example with reference to the accompanying drawings of which:
Fig. 1 is a block diagram showing the parts of an entire prosthetic hand system according to the invention;
Fig. 2 shows myoelectric signal spectra from four specific muscle contractions; and
Fig. 3 shows three useful positions of a virtual robot hand which may be achieved from the application of the system of Fig. 1 in sensing myoelectric signals from an upper limb.

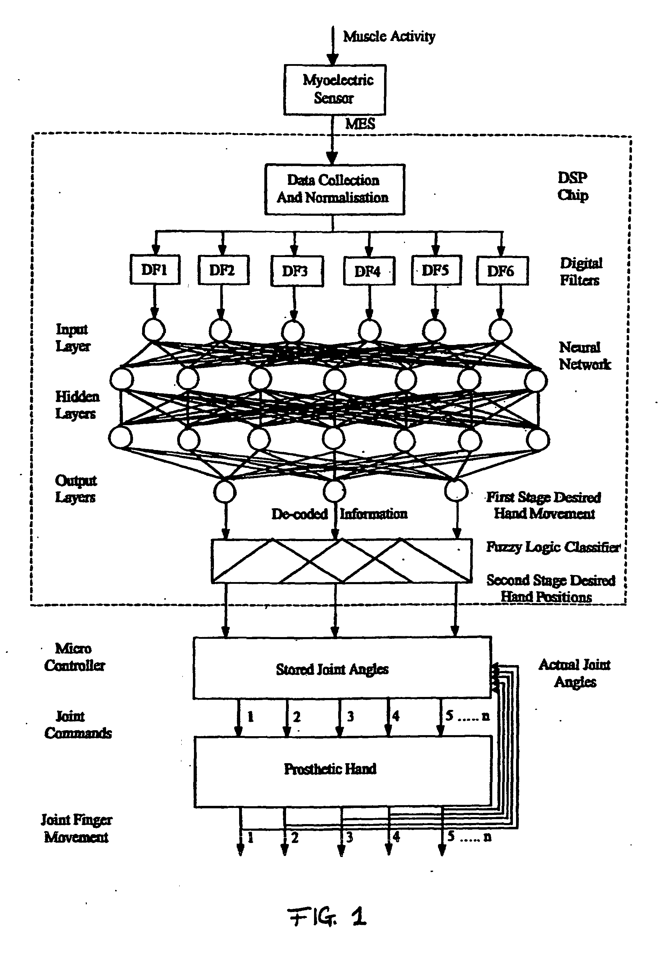

In Figure 1, the parts shown enclosed in the box defined by a dashed line correspond with the activation means of the present invention.

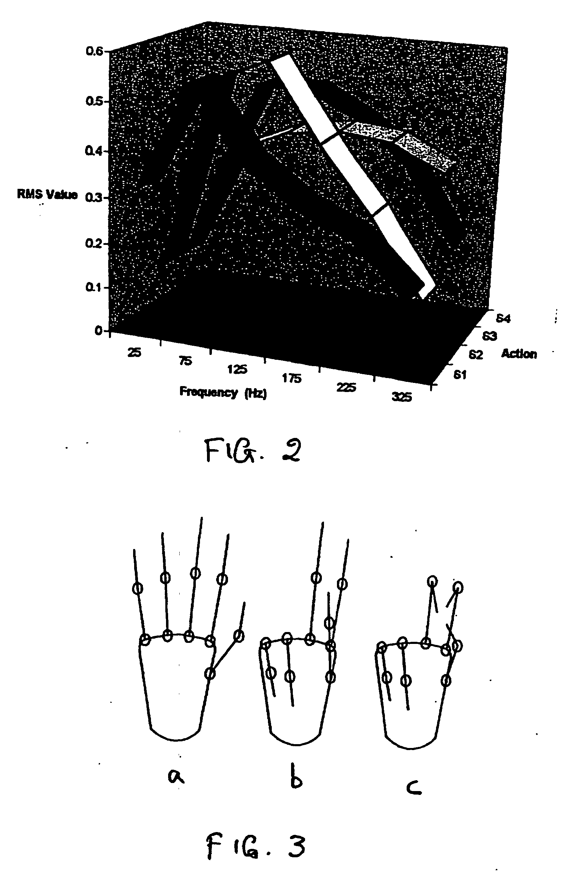

In Figure 2, the four specific positions of the hand are as follows:-
- S1:: hand relaxed with fingers and thumbs extending straight;
- S2:: ring finger bent so that tip touches the palm;
- S3:: hand relaxed with wrist bent inwards towards the palm; and
- S4:: little finger bent inwards to touch the palm.

The myoelectric signals were obtained from an individual but it has been found that, although different individuals produce their own unique spectral identities, nevertheless the overall relative pattern for a given movement or position remains reasonably consistent. In particular, the frequency components of the myoelectric signal from a single sensor carry sufficient information to discriminate between several hand positions. This information is amplitude-independent. However, the amplitude of the signals is a function of the force applied and may be used to provide a force-sensitive component to the hand movements.

With reference to Figure 3, three useful positions of a prosthetic hand are illustrated; such positions can be provided from the control system acting on the myoelectric signal spectrum of Figure 2 and are not intended necessarily to replicate the natural movements for a given myolelectric signal but rather to provide useful functionally-equivalent positions which can be reliably controlled using simple muscle movements of the upper limb. The positions shown are a) hand relaxed; b) parallel grasp and c) chuck grasp.

## Claims

1. A prosthetic limb system comprising a prosthetic limb and activation means therefor attachable to an amputee stump, in which the activation means comprises means for sensing myoelectric signals generated at the stump surface by specified muscles, means for filtering the sensed signals, means for inputting the filtered signals to an artificial neural network and means within said neural network for classifying input signals with muscle contractions representative of desired digit movements; and the prosthetic limb comprises servo-drive means to enable digits thereof to perform specified mechanical movements, said classified signals being operatively connected to said servo-drive means to enable one or more of said digits to perform an actual movement on receipt of an appropriate desired output signal from said artificial neural network, **characterised in that** the filtering means comprises bandpass filters operating within specified frequency ranges between 0 and 1000 Hz.

2. A system according to claim 1, in which the prosthetic limb comprises a prosthetic hand and the digits thereof are an artificial thumb and at least one artificial finger.

3. A system according to claim 1 or claim 2, in which the artificial neural network comprises interconnected input, hidden and output layers and is trained to recognise and react to specific patterns.

4. A system to any preceding claim, in which the frequency components of the myoelectric signal from a single sensing means carry information sufficient to enable the activation means to discriminate between several hand positions.

5. A system according to any preceding claim, in which the classified signals are passed to a cognition system comprising a fuzzy logic classifier including a rule-based system using fuzzy inference techniques for decision making.

6. A method for controlling a prosthetic limb, the method comprising the use of sensing means for measuring myoelectric signals generated by a specified muscle at the surface of an amputee stump, digitally filtering said signals, applying said digitally filtered signals to an artificial neural network programmed to produce an output signal for a prosthetic limb representing a desired limb movement for a given input signal, and causing elements of said limb to move in response to said output signal, **characterised in that** the signals are converted from the time to the frequency domain by the use of bandpass filters operating within specified frequency ranges between 0 and 1000 Hz.

7. A method according to claim 6, in which the sensing means are disposed at selected zones of the skin of the stump, according to the muscle activity which is available within the stump and the tissue filter functions which occur within said stump.

8. A method according to claim 7, in which the frequency components of the myoelectric signal from a single sensing means carry information sufficient to enable the output signal to be representative of several limb positions.

9. A method according to any of claims 6 to 8, in which the sensed signals are amplified and normalised and are preferably filtered and classified according to their frequency content in a perception subsystem.

10. A method according to any of claims 6 to 9, in which the frequency-domain signal are processed to calculate the RMS value of each filter output, preferably with performance standards for speed of recognition, percentage of non-recognitions and percentage of mis-recognitions.

11. A method according to any of claims 6 to 10 in which the decoded myoelectric signals from the neural network are passed to a fuzzy logic classifier in order to provide a definitive desired hand position.

12. A method according to any of claims 6 to 11 in which the definitive desired hand position is passed to a cognition system which will compare the actual finger positions with a suite of stored desired finger positions, any difference existing being used to actuate the fingers until the difference is zero.

## Patentansprüche

1. Gliederprothesensystem mit einer Gliederprothese und Aktivierungseinrichtungen für diese, das an einen Gliederstumpf eines Amputierten ansetzbar ist, wobei die Aktivierungseinrichtungen Einrichtungen zum Erfassen myoelektrischer Signale, die an der Stumpfoberfläche durch bestimmte Muskeln erzeugt werden, Einrichtungen zum Filtern der erfassten Signale, Einrichtungen zum Anlegen der gefilterten Signale an ein künstliches neuronales Netz sowie Einrichtungen innerhalb des neuronalen Netzes aufweist, um Eingangssignale mit gewünschte Bewegungen der Prothesenfinger darstellenden Muskelkontraktionen zu klassifizieren, und wobei die Gliederprothese Servoantriebseinrichtungen aufweist, in Folge deren deren Prothesenfinger vorgegebene mechanische Bewegungen ausführen kann, wobei die klassifizierten Signale betrieblich mit den Servoantriebseinrichtungen verbunden sind, um beim Eingang eines geeigneten Soll-Ausgangssignals aus dem künstlichen neuronalen Netz einen oder mehrere Prothesenfinger zur Ausführung einer tatsächlichen Bewegung zu veranlassen, **dadurch gekennzeichnet, dass** die Filtereinrichtung Bandfilter aufweist, die innerhalb vorgegebener Freuenzbereiche zwischen 0 und 1000 Hz arbeiten.

2. System nach Anspruch 1, bei dem die Gliederprothese eine Handprothese aufweist und deren Finger ein künstlicher Daumen und mindestens ein künstlicher Finger sind.

3. System nach Anspruch 1 oder 2, bei dem das künstliche neuronale Netz miteinander verbundene Eingangs-, versteckte und Ausgangsschichten aufweist und trainiert ist, bestimmte Muster zu erkennen und auf diese zu reagieren.

4. System nach einem der vorgehenden Ansprüche, bei dem die Frequenzkomponenten des myoelektrischen Signals aus einer einzigen Erfassungseinrichtung genug Informationen führen, dass die Aktivierungseinrichtung zwischen mehreren Handstellungen unterscheiden kann.

5. System nach einem der vorgehenden Ansprüche, bei dem die klassifizierten Signale an ein Erkenntnissystem gegeben werden, das einen Fuzzylogic-Klassifikator mit einem regelbasierten System aufweist, der zur Entscheidungsfindung mit Fuzzy-Inferenztechniken arbeitet.

6. Verfahren zum Steuern einer Gliederprothese durch Anwenden einer Erfassungseinrichtung zum Messen myoelektrischer Signale, die von bestimmten Muskeln an der Oberfläche eines Gliederstumpfes eines Amputierten erzeugt werden, digitales Filtern der Signale, Anlegen der digital gefilterten Signale an ein künstliches neuronales Netz, das so programmiert ist, dass es ein Ausgangssignal für einer Gliederprothese abgibt, das für ein gegebenes Eingangssignal eine Glied-Sollbewegung ausführt, und Bewirken, dass Elemente des Gliedes sich ansprechend auf das Ausgangssignal bewegen, **dadurch gekennzeichnet, dass** die Signale durch Anwenden von Bandfiltern, die in vorgegebenen Frequenzbereichen zwischen 0 und 1000 Hz arbeiten, aus dem Zeit- in den Frequenzbereich übertragen werden.

7. Verfahren nach Anspruch 6, bei dem entsprechend der im Stumpf verfügbaren Muskelaktivität und den im Stumpf auftretenden Gewebefilterfunktionen die Erfassungseinrichtungen in gewählten Bereichen der Haut des Stumpfes angeordnet werden.

8. Verfahren nach Anspruch 7, bei dem die Frequenzkomponenten des myoelektrischen Signals aus einer einzelnen Erfassungseinrichtung genug Informationen führen, dass das Ausgangssignal mehrere Gliederstellungen darstellen kann.

9. Verfahren nach einem der Ansprüche 6 bis 8, bei dem die erfassten Signale verstärkt, normalisiert und vorzugsweise gefiltert und entsprechend ihrem Frequenzgehalt in einem Erkennungs-Subsystem klassifiziert werden.

10. Verfahren nach einem der Ansprüche 6 bis 9, bei dem aus den Frequenzbereichssignalen der Effektivwert jedes Filterausgangssignals berechnet wird, und zwar vorzugsweise mit Leistungsstandards für die Erkennungsgeschwindigkeit, den prozentualen Anteil der Nichterkennungen und den prozentualen Anteil der Fehlerkennungen.

11. Verfahren nach einem der Ansprüche 6 bis 10, bei dem die dekodierten myoelektrischen Signale aus dem neuronalen Netz auf einen Fuzzy-logic-Klassifikator gegeben werden, um eine definitive Soll-Handstellung anzugeben.

12. Verfahren nach einem der Ansprüche 6 bis 11, bei dem die definitive Soll-Handstellung auf ein Erkenntnissystem gegeben wird, das die Ist- mit einer Folge von Soll-Fingerstellungen vergleicht, wobei eine vorhandene Abweichung zum Betätigen der Finger benutzt wird, bis die Abweichung null ist.

## Revendications

1. Système de membre prothétique comprenant une membre prothétique et un dispositif d'activation prévu pour être attaché au membre amputé, dans lequel le dispositif d'activation comprend un dispositif prévu pour capter les signaux myoélectriques générés à la surface du membre amputé par des muscles spécifiés, un dispositif de filtrage des signaux captés, un dispositif prévu pour entrer les signaux filtrés dans un réseau neuronal artificiel et un dispositif situé au sein dudit réseau neuronal destiné à classer les signaux entrants en fonction des contractions musculaires représentatives des mouvements de doigts désirés ; le membre prothétique comporte un dispositif de servocommande afin de permettre à ses doigts de réaliser les mouvements mécaniques spécifiques, lesdits signaux classés étant connectés de manière fonctionnelle audit dispositif de servocommande afin de permettre à un ou plusieurs desdits doigts d'effectuer un mouvement effectif à réception d'un signal de sortie désiré approprié à partir dudit réseau neuronal artificiel, **caractérisé en ce que** le dispositif de filtrage comporte des filtres à bande passante fonctionnant dans les limites de bandes de fréquences spécifiées allant de 0 à 1000 Hz.

2. Système selon la revendication 1, dans lequel le membre prothétique comprend une main prothétique et dont les doigts consistent en un pouce artificiel et au moins un autre doigt artificiel.

3. Système selon la revendication 1 ou 2, dans lequel le réseau neuronal artificiel comprend une couche d'entrée, une couche cachée et une couche de sortie interconnectées et est entraîné à reconnaître et à réagir à des schémas spécifiques.

4. Système selon l'une ou l'autre des revendications précédentes, dans lequel les composants de fréquence du signal myoélectrique provenant d'un dispositif de captage unique transportent une information suffisante pour permettre au dispositif d'activation de distinguer plusieurs positions de main.

5. Système selon l'une ou l'autre des revendications précédentes, dans lequel les signaux classés sont transmis à un système cognitif comportant un classifieur basé sur la logique floue comprenant un système expert utilisant des techniques d'interférences floues pour la prise de décision.

6. Procédé destiné à contrôler un membre prothétique, lequel comprend l'utilisation d'un dispositif de captage destiné à mesurer les signaux myoélectriques générés par un muscle spécifié à la surface du membre restant d'un amputé, à filtrer numériquement lesdits signaux, à appliquer lesdits signaux filtrés numériquement au réseau neuronal artificiel programmé afin de produire un signal de sortie destiné à un membre prothétique représentant un mouvement de membre désiré pour un signal d'entrée donné, et à provoquer le mouvement d'éléments dudit membre en réponse audit signal de sortie, **caractérisé en ce que** les signaux sont convertis du domaine temporel au domaine fréquentiel par l'utilisation de filtres à bande passante fonctionnant dans les limites de bandes de fréquences spécifiées allant de 0 à 1000 Hz.

7. Procédé selon la revendication 6, dans lequel les dispositifs de captage sont disposés au niveau de zones sélectionnées de la peau du membre amputé, en fonction de l'activité musculaire disponible dans le membre amputé et des fonctions de filtrage des tissus qui interviennent dans ledit membre amputé.

8. Procédé selon la revendication 7, dans lequel les composants de fréquence du signal myoélectrique provenant d'un dispositif de captage unique transportent une information suffisante pour permettre au signal de sortie d'être représentatif de plusieurs positions du membre.

9. Procédé selon l'une des revendications 6 à 8, dans lequel les signaux captés sont amplifiés et normalisés et sont de préférence filtrés et classés en fonction de leur contenu de fréquence dans un sous-système de perception.

10. Procédé selon l'une des revendications 6 à 9, dans lequel les signaux du domaine fréquentiel sont traités afin de calculer la valeur quadratique moyenne de chaque sortie de filtre, de préférence à l'aide des normes de performance pour la vitesse de reconnaissance, le pourcentage de non reconnaissances et le pourcentage de mauvaises reconnaissances.

11. Procédé selon l'une des revendications 6 à 10, dans lequel les signaux myoélectriques décodés provenant du réseau neuronal sont transférés vers un classifieur basé sur la logique floue en vue de fournir une position de main désirée définitive.

12. Procédé selon l'une des revendications 6 à 11, dans lequel la position de main désirée définitive est transmise à un système cognitif qui compare les positions des doigts déjà prises avec une suite de positions de doigts désirées stockées, toute existence de différences servant à actionner les doigts jusqu'à ce que la différence soit nulle.
